Europäisches Patentamt

European Patent Office

Office européen des brevets

(19)

(11) Numéro de publication: **0 065 473**
**B1**

(12) **FASCICULE DE BREVET EUROPEEN**

(45) Date de publication du fascicule du brevet: **20.11.86**

(51) Int. Cl.⁴: **A 61 N 1/42, A 61 N 5/00**

(21) Numéro de dépôt: **82430013.1**

(22) Date de dépôt: **12.05.82**

(54) Dispositif podal pour le traitement des zones-réflexes des pieds et, notamment, des troubles et affections du7s au déséquilibre rachidien.

(30) Priorité: **13.05.81 FR 8109663**

(43) Date de publication de la demande:
**24.11.82 Bulletin 82/47**

(45) Mention de la délivrance du brevet:
**20.11.86 Bulletin 86/47**

(84) Etats contractants désignés:
**AT BE CH DE FR GB IT LI LU NL SE**

(56) Documents cités:
**AU-A- 424 257**
**BE-A- 690 841**
**FR-A-1 450 093**
**FR-A-2 453 654**
**FR-A-2 477 022**
**US-A-3 848 588**

(73) Titulaire: **Bricot, Bernard**
**3, rue des Flots-Bleus**
**F-13007 Marseille (FR)**

(72) Inventeur: **Bricot, Bernard**
**3, rue des Flots-Bleus**
**F-13007 Marseille (FR)**

(74) Mandataire: **Marek, Pierre**
**32, rue de la Loge**
**F-13002 Marseille (FR)**

Le dossier contient des informations techniques présentées postérieurement au dépôt de la demande et ne figurant pas dans le présent fascicule.

Courier Press, Leamington Spa, England.

## Description

La présente invention concerne un dispositif podal pour la stimulation des zones-réflexes des pieds, notamment pour le traitement des troubles et des affections dûs au déséquilibre rachidien.

Des expérimentations récentes ont démontré que les systèmes musculaires staturaux dont font partie les muscles rachidiens sont sous l'influence de chaînes proprioceptives ascendantes dont le point de départ est podal. Puisque c'est le point d'appui normal en station érigée:

—de l'harmonie de ces différentes chaînes proprioceptives ascendantes dépend l'équilibre vertébral et la normalité staturale;

—toute perturbation de l'appui au sol se répercute plus haut par dysharmonie de stimulation de ces chaînes.

Il est donc apparu que l'on pouvait remédier à de nombreux troubles et affections dûs au déséquilibre rachidien par une action de ré-équilibration des chaînes proprioceptives ascendantes obtenue au moyen d'une stimulation de la plante des pieds où se trouvent les points de départ podaux de ces dernières.

Pour cela, on a proposé de corriger l'activation desdites chaînes à l'aide de semelles de forme particulière, mais si ces semelles ont donné des résultats encourageants dans le traitement de certains troubles, elles sont affectées d'un certain nombre d'inconvénients ou d'insuffisances. En effet:

—elles exigent un réglage très minutieux, l'épaisseur des câles devant être déterminée au 1/10 de millimètre près;

—leur surveillance doit être fréquente, car au fur et à mesure de la correction staturale, l'épaisseur des câles doit être modifiée;

—elles n'agissent qu'en appui bi-podal strict, de sorte qu'elles n'ont qu'un effet minimal dans le traitement des individus qui exercent une profession exigeant la position assise;

—de plus, les zones de stimulation sont simples ou doubles et n'intéresseraient donc qu'une ou deux chaînes proprioceptives, alors qu'il semblerait en exister d'autres ou, tout au moins, d'autres zones de stimulation viendraient "moduler" l'activation des chaînes principales.

Un but de la présente invention est donc de remédier à ces insuffisances en permettant une remise en équilibre des ensembles musculaires staturaux par ré-équilibration des chaînes proprioceptives ascendantes obtenue par stimulation des zones-réflexes du pied et, plus particulière-rement, des zones-réflexes de la plante des pieds où se trouvent les points de départ podaux de ces dernières, au moyen d'un flux d'énergie ondulatoire polarisée.

On a déjà proposé (FR—A—2 453 654) de traiter le corps humain au moyen d'un flux magnétique continu, pulsé ou alternatif produit par un électro-aimant ou par un aimant permanent et polarisé au moyen d'un dispositif de polarisation de la lumière constitué, par exemple, par un verre polaroïde.

Outre le fait que cette méthode thérapeutique et l'appareil conçu pour sa mise en oeuvre n'ont pas été prévus pour la stimulation des zones-réflexes du pied, ils ne conviennent pas à un tel traitement. En effet, des expérimentations ont démontré que l'application d'un flux magnétique polarisé produit par un électro-aimant ou par un aimant permanent unipolaire ordinaire sur les zones-réflexes de la plante des pieds provoquait trés vite l'apparition de signes d'intoxication magnétique avec une aggravation de certaines douleurs et l'apparition de douleurs différentes. Il a en outre été établi que certains champs magnétiques pouvaient être toxiques pour les organismes vivants. On ne peut donc envisager une stimulation permanente des zones-réflexes à l'aide de la méthode et de l'appareil susmentionnés pour le traitement des troubles de la statique nécessitant une correction permanente (scoliose, syphose, etc.). Finalement, on peut conclure que loin de favoriser la ré-équilibration des chaînes proprioceptives ascendantes, l'application, sur les zones-réflexes du pied, d'un flux magnétique polarisé engendré par un électroaimant ou par un aimant permanent ordinaire, peut aggraver le déséquilibre desdites chaînes, notamment en raison d'une intensité trop forte.

L'invention a pour objet un dispositif podal permettant d'appliquer un flux d'énergie polarisée tel qu'un flux magnétique polarisé d'intensité non toxique sur les zones-réflexes des pieds lequel entraîîne une ré-équilibration des chaînes proprioceptives ascendantes assurant une remise en équilibre des ensembles musculaires staturaux, sans provoquer l'apparition de signes d'intoxication magnétique en cours et en fin de traitement.

Le dispositif selon l'invention est defini dans la revendication 1 jointe.

Outre les avantages susmentionnés, le dispositif d'application podale selon l'invention a permis de constater, aprés quelques mois d'expérimentation à des cas de lésions non fixés:

—un ré-équilibre instantané du rachis, avec disparition des désordres rachidiens;

—une réharmonisation des ceintures scapulaires et lombaires qui s'équilibrent dans tous les plans;

—une réharmonisation des courbures rachidiennes;

—une disparition de tous les blocages vertébraux;

—une disparition de dérangements intervertébraux mineurs;

—une ouverture des trous de conjugaison;

—une disparition des douleurs;

—une augmentation de la vascularisation dans différents territoires, par libération des racines nerveuses;

—une disparition des douleurs provoquées et des douleurs projetées;

—une amélioration des peturbations fonctionnelles en rapport avec la statique.

En outre, les champs ou radiations terrestres tels que le champs magnétique terrestre étant des champs ou radiations naturels, leur application aux organismes vivants n'entraîne l'apparition d'aucun signe d'intoxication magnétique ou autre.

Les dessins annexés illustrent, à titre d'exemples non limitatifs, des formes d'exécution intéressantes de dispositifs permettant d'appliquer une énergie réticulée sur le dessous des pieds. Dans ces dessins:

La figure 1 est une vue en perspective éclatée d'une semelle médicale permettant d'appliquer une énergie réticulée sur la totalité du dessous du pied.

La figure 2 est une vue en plan de cette semelle.

La figure 3 est une vue en coupe selon la ligne 3—3 de la figure 2.

La figure 4 est une vue en plan d'une variante d'exécution de la semelle médicale selon l'invention.

La figure 5 est une vue en coupe longitudinale suivant la ligne 5—5 de la figure 4.

La figure 6 est une vue en coupe transversale d'un piedestal permettant l'application d'un flux d'énergie ondulatoire polarisée.

La figure 7 est une vue en plan de ce piedestal.

La figure 8 est une vue en plan dudit piedestal dont le fond portant les polariseurs, est représenté en position de retrait partiel.

La figure 9 est une vue en perspective éclatée d'un autre mode d'exécution d'une semelle médicale permettant d'appliquer une énergie magnétique polarisée sur la totalité du dessous du pied.

La figure 10 est une vue en plan de cette semelle.

La figure 11 est une vue en coupe selon la ligne 11—11 de la figure 10.

Le dispositif selon l'invention comprend un polariseur connu en soi, constitué par au moins deux plaques ou feuilles d'une matière ayant une orientation moléculaire ou cristalline déterminée, ces plaques ou feuilles étant superposées et disposées de façon que leurs axes de polarisation ou d'orientation soient croisés, et agencé de manière à constituer un dispositif d'appui podal, ou incorporé à un tel dispositif podal tel qu'une semelle apte à être logée dans une chaussure, un patin d'appartement, un piedestal, etc., de façon à permettre d'appliquer, su les zones-réflexes des pieds, un flux d'énergie ondulatoire polarisé engendré par les champs ou radiations terrestres tel qu'un flux magnétique polarisé engendré par le champ magnétique terrestre.

Le polariseur peut être constitué par toutes substances anisotropes ou polarisantes connues, telles que plaques polarisantes ou polaroïdes.

Dans ce cas, le polariseur est, de manière très intéressante, constitué par deux filtres polarisants linéaires superposés dont les axes de polarisation sont, de façon avantageuse, croisés à 90°. De tels filtres polarisants linéaires ou polaroïdes sont également bien connus et fréquemment utilisés en optique. Ils sont couramment constitués par des feuilles flexibles de matières plastiques contenant de cristaux microscopiques orientés à chaud sous champs électriques, lors de leur fabrication, et agissant par dichroïsme.

Il est aussi possible d'associer en superposition plus de deux plaques polarisantes ou polaroïdes dont les axes de polarisation sont, par exemple, croisés à 15°, 30°, 45°, 60°, 75°, etc., de manière à réaliser des filtres polarisants d'épaisseur variable permettant d'obtenir différentes caractéristiques de polarisation.

D'autre part, on peut employer des filtres polarisants linéaires ou des filtres polarisants circulaires.

Lorsque le polariseur est constitué de deux plaques polarisantes ou polaroïdes dont les axes sont croisés à 90°, les axes de polarisation desdits polaroïdes ont avantageusement une orientation de 45° par rapport à l'axe du pied. Toutefois, il ne s'agit pas d'un impératif absolu, les axes de polarisation des polaroïdes pouvant être croisés en formant, entre eux, des angles différents (par exemple des angles de 15°, 30°, 45°, 60°, 75°) et pouvant avoir, par rapport à l'axe du pied, une orientation différente de 45° (par exemple des angles de 30°—60°, ou des angles de 30°—45°—60°, ou des angles de 15°—30°—45°—60°—75°).

Le polariseur peut aussi être constitué par des feuilles de métal non magnétique fortement laminé ou autres substances fortement laminées ayant les mêmes propriétés sur les flux magnétiques que les filtres polaroïdes, en raison de leur orientation moléculaire ou cristalline. En cas d'emploi de deux ou plus de deux feuilles superposées de métal ou autre matériau fortement laminé, les axes d'orientation de ces feuilles (sens du laminage) peuvent être croisés de la manière indiquée ci-dessus pour les filtres polaroïdes.

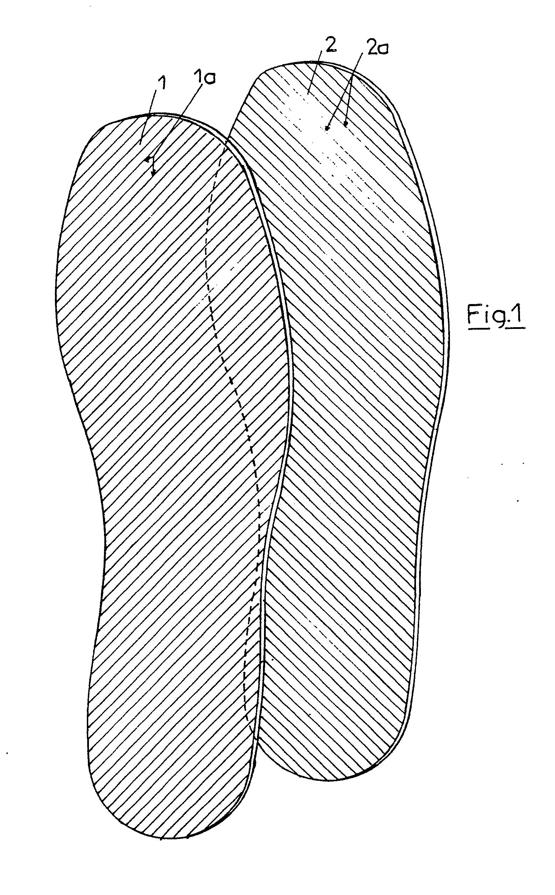

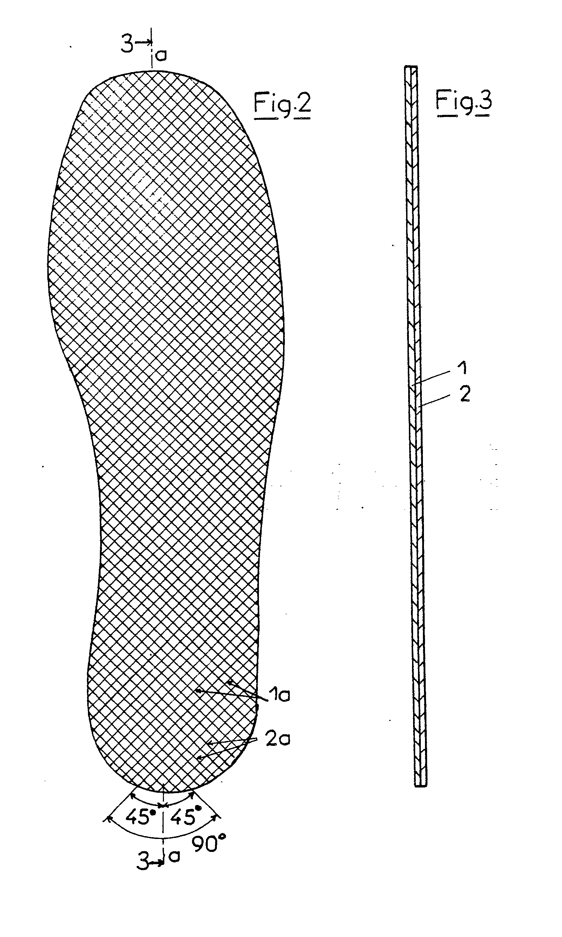

Le polariseur constitué par des plaques polarisantes souples ou polaroïdes peut être découpé à la forme du dessous des pieds pour constituer un patin ou une semelle souple destinée à être logée dans une chaussure, par exemple en cas de traitement nécessitant une lonque application de flux d'énergie ondulatoire polarisée. Ainsi, comme le montrent les figures 1 à 3, le polariseur est constitué par un patin ou semelle composée de deux filtres polarisants linéaires superposés 1 et 2 dont les axes de polarisation schématisés, respectivement, par les lignes parallèles 1a et par les lignes parallèles 2a sont croisés à 90°. D'autre part, selon cet exemple, les axes de polarisations 1a, 2a des polaroïdes croisés à 90° ont une orientation de 45° par rapport à l'axe longitudinal a—a de la semelle (figure 2), c'est-à-dire par rapport à l'axe du pied lorsque la semelle est placée sous ce dernier. La semelle ou le patin ainsi exécutés peuvent être logés dans une enveloppe protectrice réalisée dans une matière neutre, appropriée à leur mode d'utilisation, telle que cuir, feutre, tissus, etc. (non représentée).

Il peut être souhaitable de stimuler certaines zones-réflexes seulement de la plante du pied. Dans ce cas, le dispositif polariseur a une surface plus réduite que celle du dessous du pied et il est incorporé à une semelle exécutée à l'aide de matériaux traditionnels, à un emplacement correspondant à la région où se trouve(nt) la ou les zones-réflexes à traiter.

Par exemple, compte tenu du fait que les points de départ podaux des principales chaînes proprioceptives ascendantes agissant sur les ensembles musculaires staturaux se trouvent dans la région de la voûte plantaire, le dispositif polariseur peut avoir une dimension réduite et être incorporé, suivant tout procédé convenable, dans la zone médiane d'un patin ou d'une semelle exécutée de façon connue en cuir, peau, tissus, feutre, liège, ou autres matériaux, pouvant se positionner dans une chaussure.

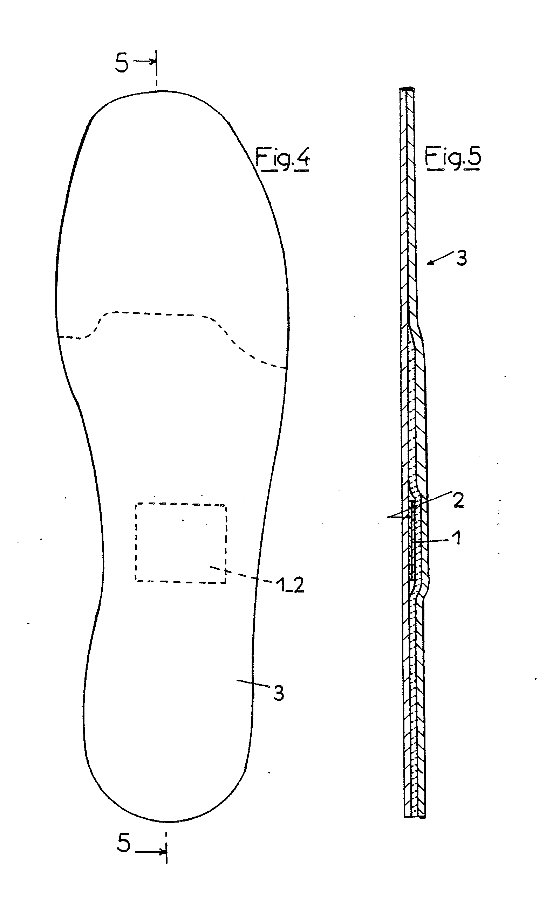

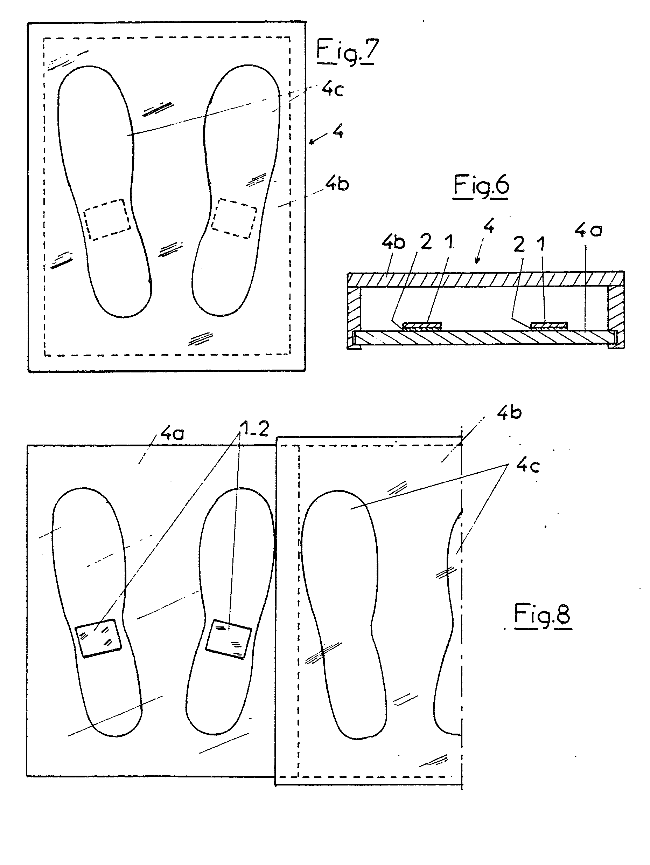

Les figures 4 et 5 montrent un mode d'exécution de ce genre suivant lequel le polariseur constitué de deux polaroïdes superposés 1, 2 est incorporé dans la partie médiane d'un patin ou semelle 3. Bien entendu, il peut aussi être placé à tout autre endroit convenable de la semelle ou patin, suivant l'emplacement de la zone-réflexe qu'il est nécessaire de stimuler.

Selon un autre mode d'exécution, on dispose deux polariseurs ayant sensiblement la forme du dessous des pieds ou des dimensions plus réduites, sur le plateau supérieur ou, de préférence, sur le fond d'un piedestal lequel peut être disposé devant le lavabo équipant une salle de bain, de façon que l'utilisateur puisse y stationner commodément et régulièrement chaque fois qu'il fait sa toilette, c'est-à-dire sans contrainte et sans perte de temps, dans le cas où le traitement prévoit des applications de flux magnétique polarisé ou autre flux d'énergie ondulatoire polarisée de durée réduite. Une réalisation de ce genre est montée aux figures 6 à 8. Suivant ce mode d'exécution, deux polariseurs constitués, chacun, de deux polaroïdes superposés 1, 2 sont installés sur le fond 4a d'un piedestal 4 formé par un boîtier creux dont ledit fond ou le plateau supérieur 4b peut être amovible ou articulé.

Sur le dessus du plateau supérieur 4b, sont figurées les empreintes 4c ou dessins de deux pieds, lesquelles se trouvent placées au-dessus des polariseurs portés par le fond 4a. De la sorte, il suffit de monter sur le piedestal 4 et de poser les pieds sur les empreintes ou dessins 4c pour que le flux d'énergie ondulatoire engendré par les champs ou radiations terrestres soit polarisé et appliqué sur les zone-réflexes du pied à traiter.

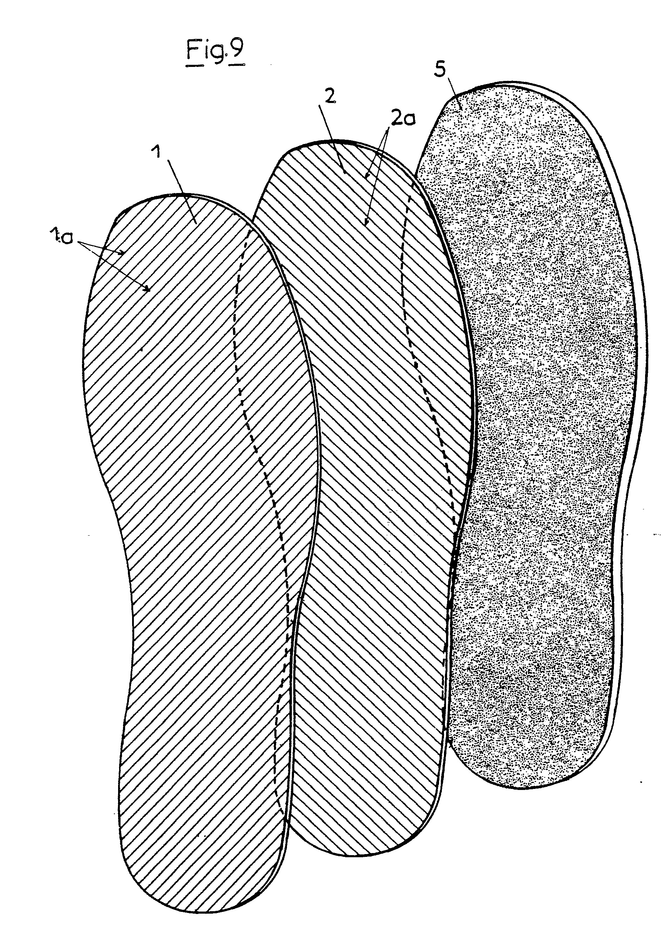

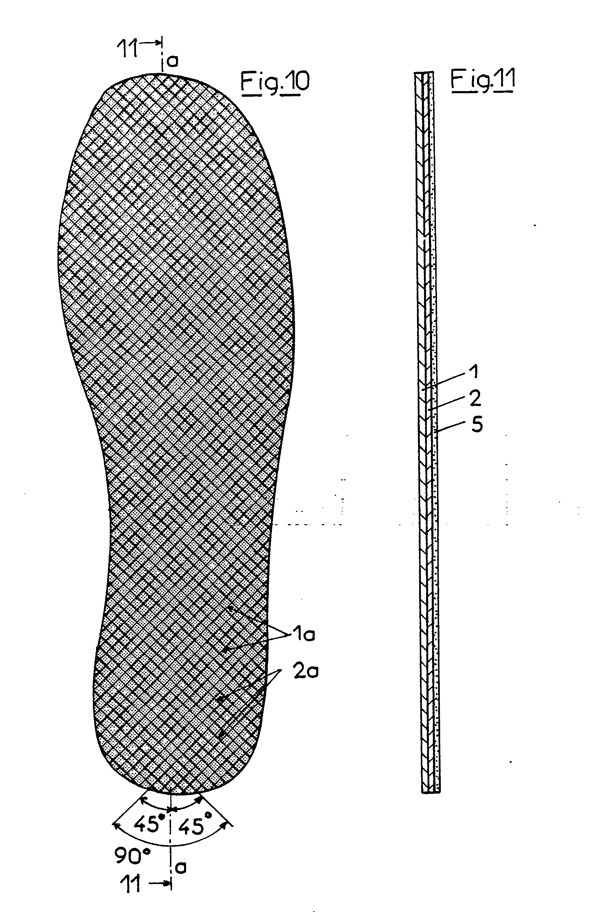

Dans le cas de douleurs fonctionnelles minimes, sans troubles importants de la statique (dorsalgies minimes, lombalgies, petites contractures musculaires, etc.) nécessitant l'application d'un flux magnétique polarisé renforcé durant des périodes quotidiennes relativement courtes, on prévoit de placer une plaque magnétique anisotrope et multipolaire au-dessous du polariseur, cette plaque magnétique ayant des formes et dimensions correspondant à celles dudit polariseur superposé. Ladite plaque magnétique anisotrope est avantageusement constituée par une feuille de caoutchouc aimanté de faible épaisseur. De tels aimants permanents flexibes et anisotropes sont bien connus. Ils sont, par exemple, composés de poudre de ferrite magnétique et d'un liant élastomère, et ils présentent une aimantation multipolaire sur l'une de leurs faces. Un mode d'exécution de ce genre est représenté aux figures 9 à 11.

Suivant ce mode d'exécution, le dispositif est constitué par une semelle médicale destinée à couvrir tout le dessous du pied. Ce dispositif comprend une feuille magnétique anisotrope et multipolaire 5 ayant la forme d'une semelle et au-dessus de la face aimantée de laquelle est disposé un polariseur compsé de deux filtres polarisants linéaires ou polaroïdes 1, 2 de forme identique à celle de ladite feuille et dont les axes de polarisation, respectivement 1a, 2a sont croisés à 90° et ont une orientation de 45° per rapport à l'axe longitudinal a—a de ladite semelle, et, par conséquent, par rapport à l'axe du pied lorsque cette dernière est utilisée. Dans ce cas, les feuilles ou plaques souples superposées 5, 1, 2 constituant la semelle médicale sont assemblées de toute manière connue. Elles peuvent, par exemple, être logées dans une enveloppe protectrice neutre et souple, ou être unies par leur bord au moyen de toute colle ou enrobage périphérique approprié.

Bien entendu, ce dispositif producteur de flux magnétique polarisé peut avoir des dimensions plus réduites de façon à pouvoir être incorporé à tout emplacement convenable d'un patin ou d'une semelle exécutée de façon classique. Il peut aussi être installé sur le plateau supérieur ou à l'intérieur d'un piedestal, comme indiqué précédemment.

On précise que, dans l'exposé qui précède et dans les revendications, le terme "ondulatoire" utilisé pour qualifier l'énergie polarisée, doit être compris comme ayant la signification suivante: "qui a le caractère de l'onde".

**Revendications**

1. Dispositif pour la stimulation des zones-réflexes des pieds, notamment pour le traitement des troubles et affections dûs au déséquilibre rachidien, caractérisé par le fait qu'il comprend un polariseur (1—2) constitué par au moins deux plaques ou feuilles (1, 2) d'une matière ayant une orientation moléculaire ou cristalline déterminée, ces plaques ou feuilles étant superposées et disposées de façon que leurs axes de polarisation ou d'orientation soient croisés, ledit polariseur ainsi constitué étant en outre agencé de manière à constituer un dispositif d'appui podal, ou incorporé à un tel dispositif d'appui podal.

2. Dispositif selon la revendication 1, caractérisé en ce que le polariseur est constitué par au moins deux polaroïdes croisés.

3. Dispositif suivant la revendication 1, caractérisé en ce que le polariseur est constitué par au moins deux feuilles de matière fortement laminée dont les axes d'orientation sont croisés.

4. Dispositif selon l'une des revendications 2 ou 3, caractérisé en ce que les axes de polarisation, (1a, 2a) des polaroïdes (1, 2) ou les axes d'orientation des feuilles de matière fortement laminée, sont croisés à 90°.

5. Dispositif suivant l'une quelconque des revendications 1 à 4, caractérisé en ce que les axes de polarisation (1a, 2a) des polaroïdes croisés (1, 2) ou les axes d'orientation des feuilles de matière fortement laminée, forment un ou des angles par rapport à l'axe longitudinal de la plante des pieds.

6. Dispositif selon la revendication 5, caractérisé en ce que les axes de polarisation (1a, 2a) des deux polaroïdes (1, 2) constituant le polariseur formé d'une paire de polaroïdes superposés, ou les axes d'orientation des deux feuilles de matière fortement laminée composant le polariseur formé d'une paire de telles feuilles, on une orientation de 45° par rapport à l'axe longitudinal (a—a) dudit dispositif déterminé par l'axe longitudinal de la plante du pied.

7. Dispositif selon l'une quelconque des revendications 1 à 6, caractérisé en ce que le polariseur (1, 2) est disposé au-dessus d'une plaque ou feuille magnétique (5) anisotrope et à aimantation multipolaire.

8. Dispositif suivant l'une quelconque des revendications 1 à 7, caractérisé en ce qu'il est constitué par un patin ou par une semelle dont les différents éléments superposés (1, 2 ou 1, 2, 5) couvrent la totalité de la surface du dessous du pied.

9. Dispositif selon l'une quelconque des revendications 1 à 7, caractérisé en ce que les différents éléments superposés (1, 2 ou 1, 2, 5) qui le composent ont une dimension plus réduite que celle de la surface du dessous du pied et sont incorporés à une semelle (3) réalisée à l'aide de matériaux connus, à un emplacement correspondant à la région où se trouve(nt) la ou les zones-réflexes à traiter de la plante du pied.

10. Dispositif suivant l'une quelconque des revendications 1 à 7, caractérisé en ce que les différents éléments superposés (1, 2 ou 1, 2, 5) qui le composent, sont installés dans le fond (4a) d'un piedestal (4) au-dessous d'emplacements (4c) destinés au positionnement des pieds de l'utilisateur et figurés sur le plateau supérieur (4b) dudit piedestal disposé à distance dudit fond.

**Patentansprüche**

1. Vorrichtung zur Stimulierung der Reflexzonen der Füße, insbesondere zur Behandlung von von Rückgratverunstaltungen verursachten Störungen und Leiden, dadurch gekennzeichnet, daß sie einen Polarisator (1—2) aufweist, der aus mindestens zwei Platten oder Folien (1, 2) aus einem Material zusammengesetzt ist, das eine bestimmte molekulare oder kristalline Orientierung hat, wobei diese Platten oder Folien übereinandergelegt und so angeordnet sind, daß ihre Polarisierungs- oder Orientierungsachsen gekreuzt sind, wobei der so gebildete Polarisator außerdem so eingerichtet ist, daß er eine Fußstützvorrichtung bildet oder in einer solchen Fußstütze integriert ist.

2. Vorrichtung nach Anspruch 1, dadurch gekennzeichnet, daß der Polarisator aus mindestens zwei gekreuzten Polaroiden besteht.

3. Vorrichtung nach Anspruch 1, dadurch gekennzeichnet, daß der Polarisator aus mindestens zwei Folien eines stark gewalzten Materials besteht, deren Orientierungsachsen gekreuzt sind.

4. Vorrichtung nach einem der Ansprüche 2 oder 3, dadurch gekennzeichnet, daß die Polarisierungsachsen (1a, 2a) der Polaroide (1, 2) oder die Orientierungsachsen der aus stark gewalztem Material bestehenden Folien bei 90° gekreuzt sind.

5. Vorrichtung nach einem der Ansprüche 1 bis 4, dadurch gekennzeichnet, daß die Polarisierungsachsen (1a, 2a) der gekreuzten Polaroide (1, 2) oder die Orientierungsachsen der aus stark gewalztem Material bestehenden Folien im Verhältnis zur Längsachse der Fußsohle einen oder mehrere Winkel bilden.

6. Vorrichtung nach Anspruch 5, dadurch gekennzeichnet, daß die Polarisierungsachsen (1a, 2a) der beiden Polaroide (1, 2), welche den aus einem Paar übereinandergelegten Polaroiden gebildeten Polarisator bilden, oder die Orientierungsachsen der beiden aus stark gewalztem Material bestehenden Folien, woraus der aus einem Paar solcher Folien gebildete Polarisator besteht, eine Orientierung von 45° im Verhältnis zur Längsachse (a—a) der besagten Vorrichtung haben, welche durch die Längsachse der Fußsohle bestimmt ist.

7. Vorrichtung nach einem der Ansprüche 1 bis 6, dadurch gekennzeichnet, daß der Polarisator (1, 2) über einer anisotropen und mit mehrpoliger Magnetisierung ausgestatteten Magnetplatte oder -folie (5) angeordnet ist.

8. Vorrichtung nach einem der Ansprüche 1 bis 7, dadurch gekennzeichnet, daß sie aus einer Fußplatte oder Sohle besteht, deren verschiedene übereinandergelegte Elemente (1, 2 oder 1, 2, 5) gesamte Unterseite des Fußes bedeckt.

9. Vorrichtung nach einem der Ansprüche 1 bis 7, dadurch gekennzeichnet, daß die verschiedenen übereinandergelegten Elemente (1, 2 oder 1, 2, 5) aus denen sie zusammengesetzt ist, eine kleinere Größe haben als die Unterseite des Fußes und in einer aus bekanntem Material hergestellten Sohle (3) an einer Stelle eingebaut sind, welche dem Bereich entspricht, wo sich die zu behandelnde(n) Reflexzone(n) der Fußsohle befindet(n).

10. Vorrichtung nach einem der Ansprüche 1 bis 7, dadurch gekennzeichnet, daß die verschiedenen übereinandergelegten Elemente (1, 2 oder 1, 2, 5) aus denen sie besteht, im Boden (4a) eines Sockels (4) unterhalb der Stellen (4c) installiert sind, die zur Postionierung der Füße des Benützers dienen und die auf der oberen Platte (4b) des in einer gewissen Entfernung vom Boden angeordneten Sockels abgebildet sind.

**Claims**

1. Device for the stimulation of the reflex zones of the feet, especially for the treatment of disorders und ailments caused by rachidian unbalance, characterised by the fact that it comprises a polariser (1—2) constituted by at least two plates or sheets (1, 2) of a material having a predetermined molecular or crystalline orientation, these plates or sheets being superposed and arranged in such a manner that their axes of polarisation or of orientation are crossed, the said polariser thus formed being furthermore made in such a manner as to constitute a pedal support device, or incorporated in such a pedal support device.

2. Device according to claim 1, characterised in that the polariser is constituted by at least two crossed polariser elements.

3. Device according to claim 1, characterised in that the polariser is constituted by at least two sheets of strongly laminated material the axes of orientation of which are crossed.

4. Device according to one of claims 2 or 3, characterised in that the axes of polarisation (1a, 2a) of the polariser elements (1, 2) or the axes of orientation of the sheets of strongly laminated material, are crossed at 90°.

5. Device according to any one of claims 1 to 4, characterised in that the axes of polarisation (1a, 2a) of the crossed polariser elements (1, 2) or the axes of orientation of the sheets of strongly laminated material form one or more angles with respect to the longitudinal axis of the sole of the feet.

6. Device according to claim 5, characterised in that the axes of polarisation (1a, 2a) of the two polariser elements (1, 2) constituting the polariser formed by a pair of superposed polariser elements, or the axes of orientation of the two sheets of strongly laminated material composing the polariser formed by a pair of such sheets, have an orientation of 45° with respect to the longitudinal axis (a—a) of the said device determined by the longitudinal axis of the sole of the foot.

7. Device according to any one of claims 1 to 6, characterised in that the polariser (1, 2) is placed above an anisotropic magnetic plate or sheet (5) of multipolar magnetisation.

8. Device according to any one of claims 1 to 7, characterised in that it is constituted by an insole or by a sole of which the different superposed elements (1, 2 or 1, 2, 5) cover the whole of the undersurface of the foot.

9. Device according to any one of claims 1 to 7, characterised in that the different superposed elements (1, 2 or 1, 2, 5) which compose it have a smaller dimension than that of the undersurface of the foot and are incorporated in a sole (3) made of known materials, at a position corresponding to the region where there are to be found the reflex zone or zones to be treated of the sole of the foot.

10. Device according to any one of claims 1 to 7, characterised in that the different superposed elements (1, 2 or 1, 2, 5) which compose it, are installed in the base (4a) of a pedestal (4) below emplacements (4c) serving for the positioning of the feet of the user and represented on the upper plate (4b) of the said pedestal disposed at a distance from the said base.

Fig.1

0 065 473

Fig.2

Fig.3

45° | 45°

90°

2

0 065 473

Fig.4

Fig.5

5

5

3

1_2

3

2

1

3

0 065 473

Fig.7

Fig.6

Fig.8

4

Fig.9

11 →

a

Fig.10

45° | 45°

90°

11 →

a

1a

2a

Fig.11

1
2
5